# DEMANDE DE BREVET EUROPEEN

(11) **EP 1 240 887 A1**
(43) Date de publication de la demande: **18.09.2002**
(21) Numéro de dépôt: 02290464.3
(22) Date de dépôt: 26.02.2002
(51) Int. Cl.: A61K 7/06, A61K 7/48

(54) **Dispositif aerosol contenant une composition capillaire comprenant un polysaccharide greffe par un polysiloxane**

(30) Priorité: 13.03.2001 FR 0103407
(71) Demandeur: L'OREAL, 75008 Paris (FR)
(72) Inventeur: Giroud, Franck, 92110 Clichy (FR); Dupuis, Christine, 75018 Paris (FR)
(74) Mandataire: Bourdeau, Françoise

(57) **Abrégé**

L'invention a pour objet un dispositif aérosol contenant une composition cosmétique capillaire comprenant au moins un polysaccharide greffé par un polysiloxane, Elle vise également un procédé cosmétique capillaire, en particulier un procédé de fixation et/ou de maintien de la coiffure mettant en oeuvre ce dispositif aérosol.

## Description

L'invention a pour objet un dispositif aérosol contenant une composition cosmétique capillaire comprenant au moins un polysaccharide greffé par un polysiloxane. Elle vise également un procédé cosmétique capillaire, en particulier un procédé de fixation et/ou de maintien de la coiffure mettant en oeuvre ce dispositif aérosol.

Parmi les produits capillaires pour la mise en forme et/ou le maintien de la coiffure les plus répandus sur le marché de la cosmétique, on peut citer les compositions à pulvériser essentiellement constituées d'une solution le plus souvent alcoolique ou aqueuse et d'un ou plusieurs matériaux, généralement des résines polymères, dont la fonction est de former des soudures entre les cheveux, appelés encore matériaux fixants, en mélange avec divers adjuvants cosmétiques. Cette solution est généralement conditionnée soit dans un récipient aérosol approprié mis sous pression à l'aide d'un propulseur, soit dans un flacon pompe.

Les matériaux fixants sont généralement des polymères fixants, c'est à dire des polymères filmogènes solubles dans l'eau et dans l'alcool, tels que la polyvinylpyrrolidone, les copolymères de vinyipyrrolidone/acétate de vinyle, décrits notamment dans les brevets US 3 929 735 et US 3 770 683, les copolymères d'acétate de vinyle/acide crotonique, les résines acryliques anioniques ou amphotères. Ces matériaux permettent d'obtenir facilement l'effet fixant, en revanche, après brossage ou peignage, les cheveux présentent dans les conditions usuelles de laquage un aspect raidi et un toucher rêche, voire collant ainsi qu'un poudrage après séchage.

Par ailleurs, la qualité de la pulvérisation obtenue à partir d'un dispositif aérosol est parfois insuffisante. Avec certaines compositions cosmétiques, il est, par exemple, difficile d'ajuster à volonté la taille des gouttelettes ou le débit de la pulvérisation.

Ces inconvénients sont liés à plusieurs paramètres, parmi lesquels on peut citer la nature du ou des polymères fixants, la nature des soudures ou la composition en gaz propulseurs. Pour remédier à ces inconvénients on peut donc agir sur plusieurs paramètres, sans toutefois diminuer l'effet fixant recherché.

La Demanderesse a maintenant trouvé qu'en sélectionnant, de manière appropriée, à la fois, le polymère fixant et le gaz propulseur, il était possible de remédier aux différents problèmes énumérés ci-dessus, et en particulier d'obtenir un spray de bonne qualité en termes de diffusion et de taille des gouttelettes, de propriétés cosmétiques et de poudrage.

L'invention a pour objet un dispositif aérosol constitué par un récipient contenant une composition aérosol constituée, d'une part par une phase liquide (ou jus) contenant au moins un matériau fixant dans un solvant approprié et d'autre part par un propulseur, ainsi qu'un moyen de distribution de ladite composition aérosol, caractérisé par le fait que :
(i) le matériau fixant est un polymère à squelette de type polysaccharide greffé par des chaînons contenant au moins un polysiloxane, et
(ii) le propulseur est le diméthyléther.

Un autre objet de l'invention concerne un procédé cosmétique capillaire, en particulier un procédé de fixation et/ou de maintien de la coiffure mettant en oeuvre le dispositif aérosol.

Encore un autre objet de l'invention concerne l'utilisation de ce dispositif en cosmétique capillaire, notamment pour le maintien et/ou la mise en forme de la coiffure.

Les polymères à squelettes de type polysaccharide greffés par des chaînons contenant au moins un polysiloxane conformes à la présente invention comprennent une chaîne principale formée à partir de polysacharide(s), sur laquelle se trouve greffé, ainsi qu'éventuellement à l'une au moins de ses extrémités, au moins un chaînon contenant un polysiloxane.

Les polymères à squelettes de type polysaccharide greffés par des chaînons contenant au moins un polysiloxane conformes à la présente invention peuvent être obtenus selon tout moyen connu de l'homme de l'art, en particulier par réaction entre (i) un macromère polysiloxane de départ correctement fonctionnalisé sur la chaîne polysiloxanique et (ii) un ou plusieurs polysaccharide(s), eux-mêmes correctement fonctionnalisés par une fonction, capable de venir réagir avec le ou les groupements fonctionnels portés par ladite silicone pour former une liaison covalente.

Les polymères à squelettes polysaccharide greffés par des chaînons contenant au moins un polysiloxane conformes à l'invention sont choisis, de préférence, parmi ceux décrits dans le brevet US 6 066 727 déposé par la Société Shin-Etsu. Il s'agit de copolymères obtenus par réaction entre un polysaccharide contenant des groupements carboxyles et un polysiloxane contenant un groupement époxy terminal, dans un solvant organique, en présence éventuellement d'un catalyseur.

En particulier, les polymères à squelettes polysaccharide greffés par des chaînons contenant au moins un polysiloxane utilisés plus préférentiellement conformément à la présente invention sont susceptibles d'être obtenus conformément au procédé décrit dans le brevet US 6 066 727, c'est-à-dire en :
(a) mettant en solution, dans un solvant organique, un polysaccharide contenant des groupements carboxyles et un polysiloxane, contenant lui-même un groupement époxy terminal, 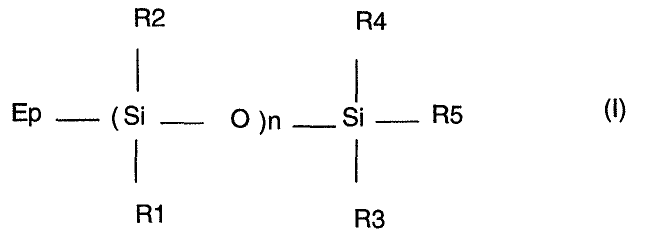 et répondant à la formule (I) de polysiloxane suivante :
   dans laquelle :
      - n est un nombre entier compris entre 3 à 500,
      - R₁, R₂, R₃, R₄ et R₅ sont choisis, indépendamment l'un de l'autre, parmi les hydrocarbures monovalents en C₁ à C₁₀ ou les hydrocarbures halogénés monovalents en C₁ à C₁₀, et
      - Ep est le 2-(3,4-époxycyclohexyl) éthyl ; et
(b) chauffant ce mélange de polysaccharides et de polysiloxanes à une température comprise entre 60 et 200 °C, de façon à faire réagir les groupements carboxyles des polysaccharides avec les groupements époxy des polysiloxanes.

Parmi des polysaccharides convenant particulièrement pour la mise en oeuvre de l'étape (a) de ce procédé, on peut citer les polysaccharides possédant des groupements carboxylique, benzoyle ou succinoyle, tels que l'hydroxypropyl méthyl cellulose phtalate, l'hydroxypropyl méthyl cellulose acétate succinate, le carboxyméthyl éthyl cellulose, le polysaccharide pullulan acétate phthalate. On préfère, notamment, l'hydroxypropyl méthyl cellulose phthalate et l'hydroxypropyl méthyl cellulose acétate succinate.

De manière avantageuse, R₁, R₂, R₃, R₄ et R₅ sont choisis, indépendamment l'un de l'autre, parmi les groupements méthyl, éthyl, propyl, butyl, cycloalkyl en C₃ à C₈, tels que les radicaux cyclopentyl ou cyclohexyl, aryles, tels que les radicaux phényl et le tolyl, aralkyls en C₃ à C₈, tels que les radicaux benzyl et phénéthyl et alcényl, tels que les groupes vinyl et allyl. Ces radicaux hydrocarbyls monovalents peuvent, éventuellement, être totalement ou partiellement substitués, en particulier, par un atome d'halogène, comme les radicaux chlorométhyl et 3,3,3-trifluoropropyl.

En tant que solvants organiques convenant spécialement bien pour la réalisation de l'étape (a) du procédé, on peut citer les cétones, comme l'acétone ou la cyclohexanone.

L'étape (b) est généralement effectuée sous agitation, éventuellement sous atmosphère inerte.

La composition comprend avantageusement, en pourcentage relatif en poids de la composition, de 0,1 à 20 % de polysaccharide greffé par des polysiloxanes, et plus préférentiellement de 0,5 à 10 % de polysaccharide.

La composition comprend avantageusement, en pourcentage relatif en poids de la composition, de 5 à 90 % de diméthyl éther, et plus préférentiellement de 10 à 50 % de ce propulseur.

Le milieu cosmétiquement acceptable est, de préférence, constitué par de l'eau ou un ou plusieurs solvants cosmétiquement acceptables ou des mélanges eau-solvants. Ces solvants sont, de préférence, des alcools en C₁-C₄, l'éthanol étant particulièrement préféré.

La composition de l'invention peut également contenir au moins un additif choisi parmi les tensioactifs anioniques, cationiques, non ioniques ou amphotères, les parfums, les filtres, les conservateurs, les protéines, les vitamines, les céramides, les polymères autres que ceux de l'invention, et en particulier les polymères fixants anioniques, amphotères ou non ioniques, les huiles végétales, minérales ou synthétiques et tout autre additif classiquement utilisé dans les compositions cosmétiques.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas ou substantiellement pas, altérées par l'addition envisagée.

Les compositions conformes à l'invention sont particulièrement adaptés pour des cheveux secs ou humides, en tant que produits de coiffage.

Les compositions conformes à l'inventions peuvent être appliquées sur la peau, les ongles, les lèvres, les cheveux, les sourcils et les cils.

L'invention va être plus complètement illustrée à l'aide de l'exemples non limitatif suivant.

Tous les pourcentages sont des pourcentages relatifs en poids par rapport au poids total de la composition et m.a. signifie matière active.

### EXEMPLE :

On réalise le dispositif aérosol conforme à l'invention contenant la composition suivante.

| | |
|---|---|
| Cellulose à greffons siliconés [1] | 3%ma |
| eau | 17% |
| Ethanol | 45% |
| Diméthyl éther | 35% |

| | |
|---|---|
| [1] Hydroxy proplyl methyl cellulose acétate succinate à greffons polydiméthylsiloxane, telle qu'elle est synthétisée dans l'exemple N°2 du brevet US 6,066,727 de la société Shin Etsu. | |

Cette composition est pulvérisée sur des cheveux eurochâtains. Les cheveux ont de bonnes propriétés cosmétiques dans les deux cas, après séchage et donnent peu de poudrage.

## Revendications

1. Dispositif aérosol constitué par un récipient contenant une composition aérosol constituée, d'une part par une phase liquide (ou jus) contenant au moins un matériau fixant dans un solvant approprié et d'autre part par un propulseur, ainsi que d'un moyen de distribution de ladite composition aérosol, **caractérisé par le fait que** :
(i) le matériau fixant est un polymère à squelette de type polysaccharide greffé par des chaînons contenant au moins un polysiloxane, et
(ii) le propulseur est le diméthyléther.

2. Dispositif selon la revendication 1, **caractérisé par le fait que** les polymères à squelettes de type polysaccharide greffés par des chaînons contenant au moins un polysiloxane comprennent une chaîne principale formée à partir de polysacharide(s), sur laquelle se trouve greffé, ainsi qu'éventuellement à l'une au moins de ses extrémités, au moins un chaînon contenant un polysiloxane.

3. Dispositif selon l'une quelconque des revendications précédentes, **caractérisé par le fait que** les que les polymères à squelettes de type polysaccharide greffés par des chaînons contenant au moins un polysiloxane sont susceptibles d'être obtenus en :
(a) mettant en solution, dans un solvant organique, un polysaccharide contenant des groupements carboxyles et un polysiloxane, contenant lui-même un groupement époxy terminal, 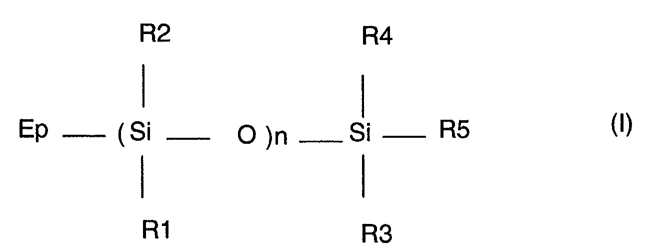 et répondant à la formule (I) de polysiloxane suivante :
dans laquelle :
- n est un nombre entier compris entre 3 à 500,
- R₁, R₂, R₃, R₄ et R₅ sont choisis, indépendamment l'un de l'autre, parmi les hydrocarbures monovalents en C₁ à C₁₀ ou les hydrocarbures halogénés monovalents en C₁ à C₁₀, et
- Ep est le 2-(3,4-époxycyclohexyl) éthyl ; et
(b) chauffant ce mélange de polysaccharides et de polysiloxanes à une température comprise entre 60 et 200 °C, de façon à faire réagir les groupements carboxyles des polysaccharides avec les groupements époxy des polysiloxanes.

4. Dispositif selon la revendication 3, **caractérisé par le fait que** pour la mise en oeuvre de l'étape (a), on utilise, en tant que polysaccharides, des polysaccharides possédant des groupements carboxylique, benzoyle ou succinoyle, tels que l'hydroxypropyl méthyl cellulose phtalalate, l'hydroxypropyl méthyl cellulose acétate succinate, le carboxyméthyl éthyl cellulose, le polysaccharide pullulan acétate phthalate.

5. Dispositif selon la revendication 3, **caractérisé par le fait que** R₁, R₂, R₃, R₄ et R₅ sont choisis, indépendamment l'un de l'autre, parmi les groupements méthyl, éthyl, propyl, butyl, cycloalkyl en C₃ à C₈, aryls, aralkyls en C₃ à C₈ et alcényl.

6. Dispositif selon l'une quelconque des revendications précédentes, **caractérisé par le fait que** la composition comprend, en pourcentage relatif en poids de la composition, de 0,1 à 20 % de polysaccharide greffé par des polysiloxanes, et plus préférentiellement de 0,5 à 10 % de ce polysaccharide.

7. Dispositif selon l'une quelconque des revendications précédentes, **caractérisé par le fait qu'**il comprend, en pourcentage relatif en poids de la composition, de 5 à 90 % de diméthyl éther, et plus préférentiellement de 10 à 50 % de ce propulseur.

8. Dispositif selon l'une quelconque des revendications précédentes, **caractérisé par le fait qu'**il contient au moins un additif choisi parmi les tensioactifs anioniques, cationiques, non ioniques ou amphotères, les parfums, les filtres, les conservateurs, les protéines, les vitamines, les céramides, les polymères autres que ceux de l'invention, et en particulier les polymères fixants anioniques, amphotères ou non ioniques, les huiles végétales, minérales ou synthétiques et tout autre additif classiquement utilisé dans les compositions cosmétiques.

9. Procédé cosmétique capillaire, en particulier un procédé de fixation et/ou de maintien de la coiffure mettant en oeuvre un dispositif selon l'une quelconque des revendications 1 à 8.

10. Utilisation d'un dispositif selon l'une quelconque des revendications 1 à 8 en cosmétique capillaire, notamment pour le maintien et/ou la mise en forme de la coiffure.
